# EUROPEAN PATENT APPLICATION

(11) **EP 3 821 898 A1**
(43) Date of publication of application: **19.05.2021**
(21) Application number: 20217061.9
(22) Date of filing: 30.04.2013
(51) Int. Cl.: A61K 35/17, C12N 5/0783, A61K 39/00, A61P 35/00, A61P 35/02

(54) **METHODS OF PRODUCING ENRICHED POPULATIONS OF TUMOR-REACTIVE T CELLS FROM TUMOR**

(30) Priority: 01.03.2013 US 201361771247 P
(62) Divisional of application: 13722922.5
(71) Applicant: The United States of America, as represented by The Secretary, Department of Health and Human Services, Bethesda, MD 20852-7660 (US)
(72) Inventor: Gros, Alena, Washington, District of Columbia 20009 (US); Rosenberg, Steven A., Potomac, Maryland 20854 (US)
(74) Representative: Maiwald Patent- und Rechtsanwaltsgesellschaft mbH

(57) **Abstract**

Methods of obtaining a cell population enriched for tumor-reactive T cells, the method comprising: (a) obtaining a bulk population of T cells from a tumor sample; (b) specifically selecting CD8⁺ T cells that express any one or more of TIM-3, LAG-3, 4-1BB, and PD-1 from the bulk population; and (c) separating the cells selected in (b) from unselected cells to obtain a cell population enriched for tumor-reactive T cells are disclosed. Related methods of administering a cell population enriched for tumor-reactive T cells to a mammal, methods of obtaining a pharmaceutical composition comprising a cell population enriched for tumor-reactive T cells, and isolated or purified cell populations are also disclosed.

## Description

### CROSS REFERENCE TO RELATED APPLICATION

This patent application claims the benefit of U.S. Provisional Patent Application No. 61/771,247, filed March 1, 2013, which is incorporated by reference in its entirety herein. This invention was made with Government support under project number ZIABC010984 by the National Institutes of Health, National Cancer Institute. The Government has certain rights in the invention.

### BACKGROUND OF THE INVENTION

Adoptive cell therapy (ACT) using tumor-reactive T cells can produce positive clinical responses in some cancer patients. Nevertheless, several obstacles to the successful use of ACT for the treatment of cancer and other diseases remain. For example, T cells isolated from a tumor may not exhibit sufficient tumor-specific reactivity. Accordingly, there is a need for improved methods of obtaining a population of tumor-reactive T cells from tumors.

### BRIEF SUMMARY OF THE INVENTION

An embodiment of the invention provides a method of obtaining a cell population enriched for tumor-reactive T cells, the method comprising: (a) obtaining a bulk population of T cells from a tumor sample; (b) specifically selecting CD8⁺ T cells that express any one or more of TIM-3, LAG-3, 4-1BB, and PD-1 from the bulk population; and (c) separating the cells selected in (b) from unselected cells to obtain a cell population enriched for tumor-reactive T cells.

Another embodiment of the invention provides a method of administering a cell population enriched for tumor-reactive T cells to a mammal, the method comprising: (a) obtaining a bulk population of T cells from a tumor sample; (b) specifically selecting CD8⁺ T cells that express any one or more of TIM-3, LAG-3, 4-1BB, and PD-1 from the bulk population; (c) separating the cells selected in (b) from unselected cells to obtain a cell population enriched for tumor-reactive T cells; and (d) administering the cell population enriched for tumor-reactive T cells to the mammal.

Still another embodiment of the invention provides a method of obtaining a pharmaceutical composition comprising a cell population enriched for tumor-reactive T cells, the method comprising: (a) obtaining a bulk population of T cells from a tumor sample; (b) specifically selecting CD8⁺ T cells that express any one or more of TIM-3, LAG-3, 4-1BB, and PD-1 from the bulk population; (c) separating the cells selected in (b) from unselected cells to obtain a cell population enriched for tumor-reactive T cells; and (d) combining the cell population enriched for tumor-reactive T cells with a pharmaceutically acceptable carrier to obtain a pharmaceutical composition comprising a cell population enriched for tumor-reactive T cells.

Another embodiment of the invention provides a cell population enriched for tumor-reactive T cells obtained by a method comprising: (a) obtaining a bulk population of T cells from a tumor sample; (b) specifically selecting CD8⁺ T cells that express any one or more of TIM-3, LAG-3, 4-1BB, and PD-1 from the bulk population; and (c) separating the cells selected in (b) from unselected cells to obtain a cell population enriched for tumor-reactive T cells for use in administering the cell population enriched for tumor-reactive T cells to a mammal.

Additional embodiments of the invention provide related populations of cells and methods of treating or preventing cancer.

### BRIEF DESCRIPTION OF THE SEVERAL VIEWS OF THE DRAWINGS

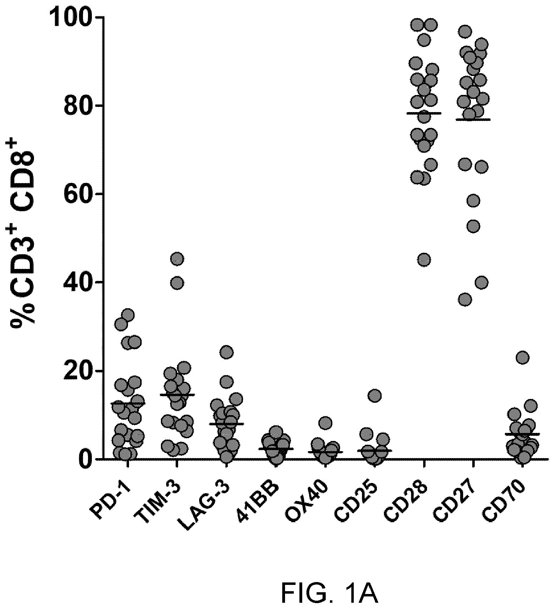

Figure 1A is a graph showing the percentage of CD3⁺/CD8⁺ cells isolated from fresh melanoma tumor samples expressing PD-1, TIM-3, LAG-3, 4-1BB, OX40, CD25, CD28, CD27, or CD70. Each dot represents one tumor.

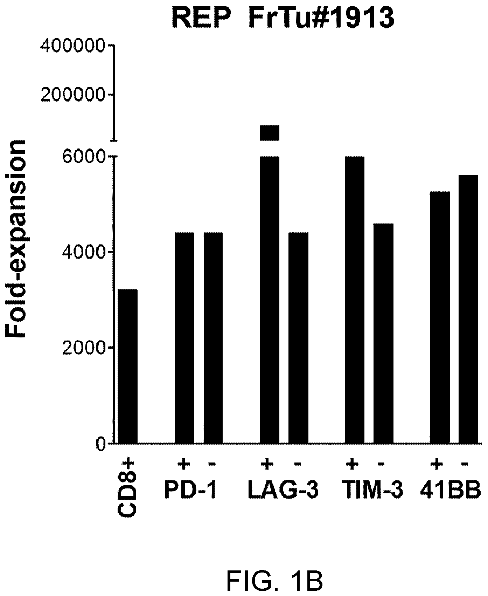

Figure 1B is a graph showing fold-expansion of the numbers of CD8⁺ cells that were isolated from a fresh melanoma tumor sample (FrTu#1913), sorted for expression of CD8, PD-1, LAG-3, TIM-3, or 4-1BB, or lack of expression of PD-1, LAG-3, TIM-3, or 4-1BB, after *in vitro* expansion (REP) for 14 days.

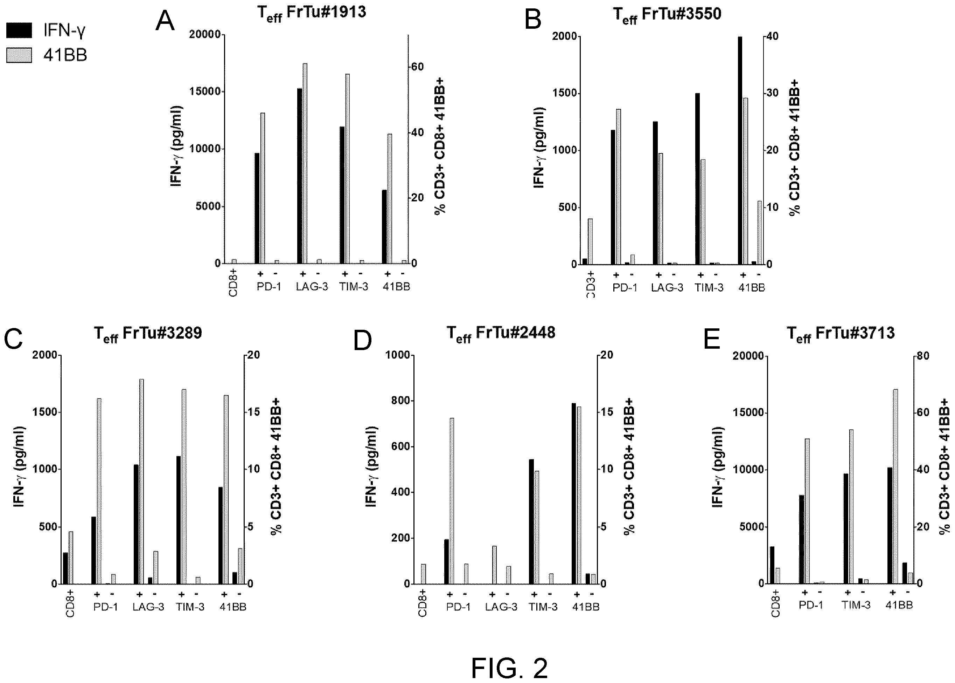

Figures 2A-2E show interferon (IFN)-gamma secretion (pg/ml) (black bars) or percentage of effector T-cells (T_{eff}) expressing CD3, CD8, and 4-1BB (grey bars) by CD8⁺ cells isolated from one of five different melanoma tumor samples (FrTu#1913 (A), FrTu#3550 (B), FrTu#3289 (C), FrTu#2448 (D), or FrTu#3713 (E)). Cells were sorted for expression of CD8, PD-1, LAG-3, TIM-3, or 4-1BB, or lack of expression of PD-1, LAG-3, TIM-3, or 4-1BB, and expanded *in vitro* for 14 days. Interferon (IFN)-gamma secretion and 4-1BB expression were assayed upon co-culture with autologous tumor cell lines.

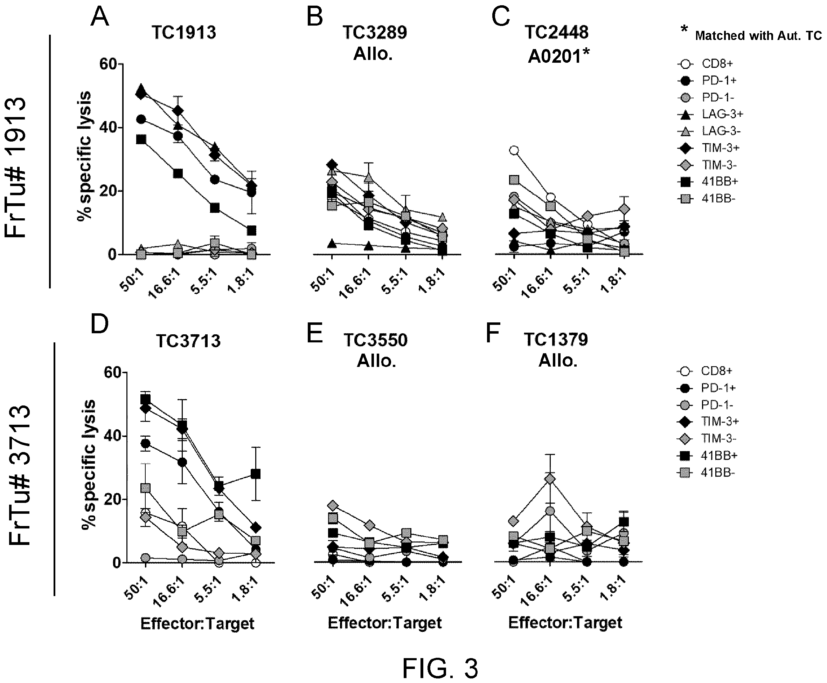

Figures 3A-3C show percent specific lysis of target tumor cell lines TC1913 (autologous) (A), TC3289 (allogeneic) (B), or TC2448 (HLA-A0201 matched) (C) by effector CD8⁺ T cells that were isolated from melanoma tumor sample FrTu#1913 and sorted for expression of CD8 (open circles), PD-1 (black circles), TIM-3 (black diamonds), LAG-3 (black triangles), or 4-1BB (black squares) or lack of expression of PD-1 (grey circles), TIM-3 (grey diamonds), LAG-3 (grey triangles), or 4-1BB (grey squares) at the effector:target ratios indicated.
Figures 3D-3F show percent specific lysis of target tumor cell lines TC3713 (autologous) (D), TC3550 (allogeneic) (E) or TC1379 (allogeneic) (F) by effector CD8⁺ T cells that were isolated from melanoma tumor sample FrTu#3713 (D-F) and sorted for expression of CD8 (open circles), PD-1 (black circles), TIM-3 (black diamonds), or 4-1BB (black squares) or lack of expression of PD-1 (grey circles), TIM-3 (grey diamonds), or 4-1BB (grey squares) at the effector:target ratios indicated.

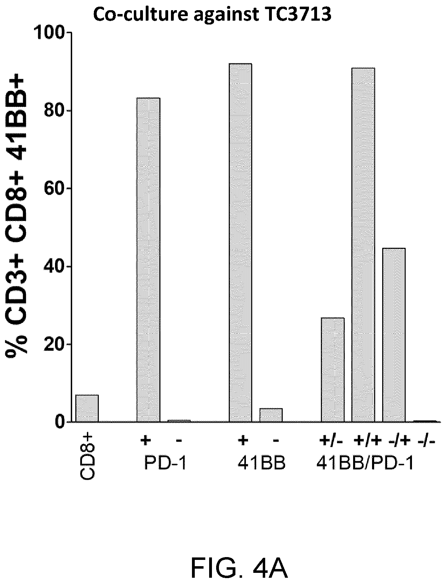

Figure 4A shows autologous tumor recognition of cells isolated from a melanoma tumor (FrTu#3713), sorted for CD8⁺, PD-1⁺ PD-1⁻, 4-1BB⁺, 4-1BB⁻, 4-1BB⁺/PD-1⁻, 4-1BB⁺/PD-1⁺, 4-1BB⁻/PD-1⁺, or 4-1BB⁻/PD-1⁻ and expanded *in vitro* for 14 days. Percentage of CD3⁺ CD8⁺ cells expressing 4-1BB upon co-culture with autologous tumor cell lines is shown.

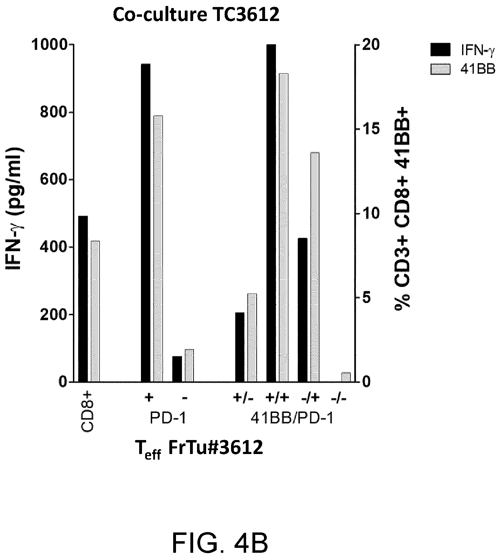

Figure 4B is a graph showing the percentage of CD3⁺CD8⁺ cells that express 4-1BB (grey bars) or secrete IFN-gamma (black bars) after being isolated from a melanoma tumor (FrTu#3612). Cells were sorted for CD8⁺, PD-1⁺, PD-1⁻, 4-1BB⁺/PD-1⁻, 4-1BB⁺/PD-1⁺, 4-1BB⁻/PD-1⁺, or 4-1BB⁻/PD-1-populations, expanded *in vitro* for 14 days and IFN-gamma secretion and 4-1BB up-regulation upon co-culture with autologous tumor cell lines is shown.

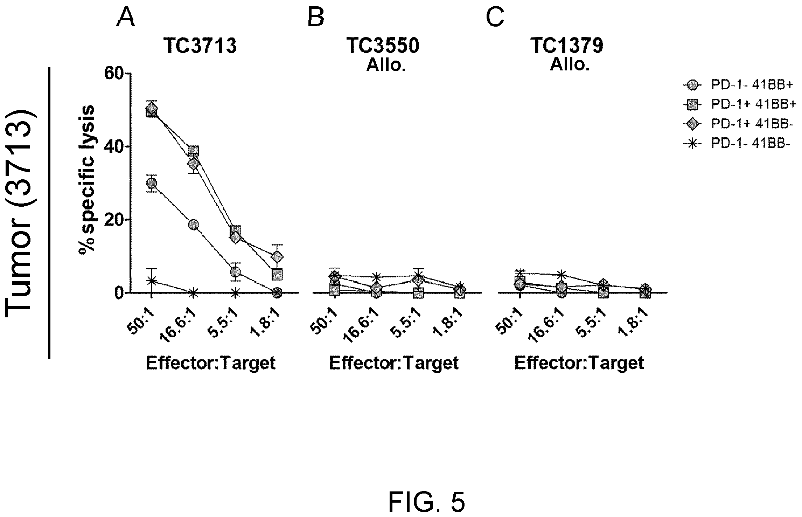

Figures 5A-5C show percent specific lysis of target tumor cell lines TC3713 (autologous) (A), TC3550 (allogeneic) (B) and TC1379 (allogeneic) (C) by effector CD8⁺ cells that were isolated from a melanoma tumor (FrTu#3713) and sorted for 4-1BB⁺/PD-1⁻ (circles)), 4-1BB⁺/PD-1⁺ (squares), 4-1BB⁻/PD-1⁺ (diamonds), or 4-1BB⁻/PD-1⁻(*) populations at the effector to target ratios indicated as measured by ⁵¹Cr release assay.

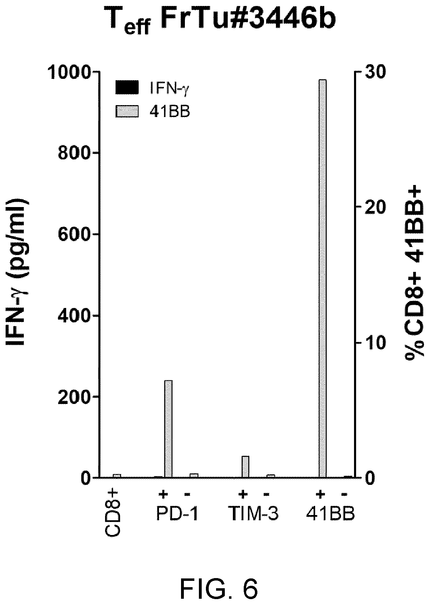

Figure 6 is a graph showing the percentage of CD8⁺ cells that express 4-1BB (grey bars) or secrete IFN-gamma (black bars) that were isolated from a gastrointestinal tumor (FrTu#3446b), sorted for CD8⁺, PD-1⁺, PD-1⁻, TIM-3⁺, TIM-3⁻, 4-1BB⁺, or 4-1BB⁻ populations and expanded for 21 days in culture. IFN-gamma and 4-1BB up-regulation upon co-culture with autologous tumor cell lines is shown.

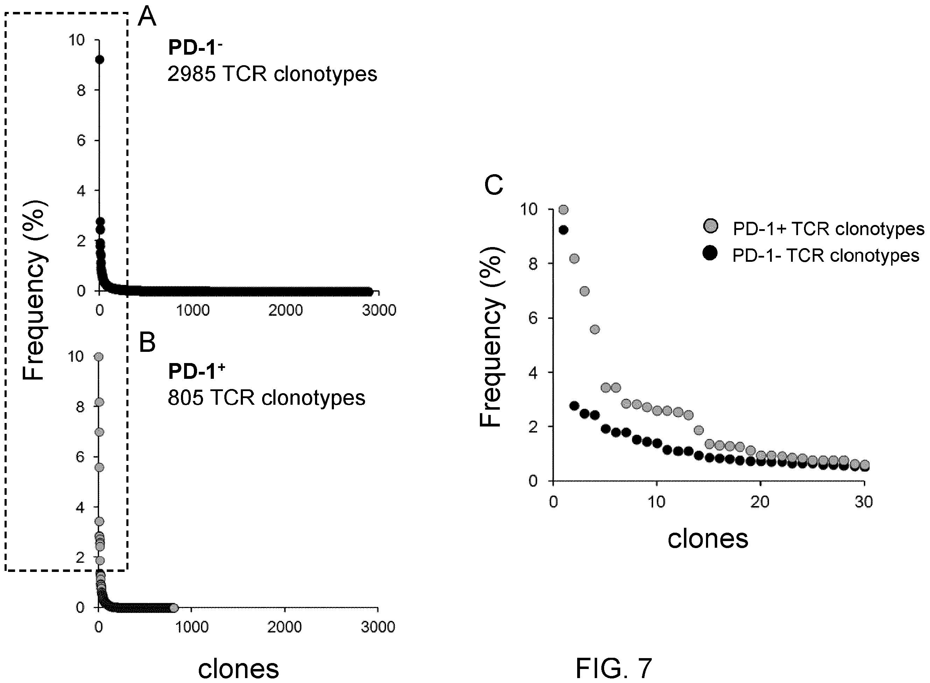

Figures 7A and 7B are graphs showing the frequency (%) of unique TCR beta chain CDR3 region amino acid sequences of sorted PD-1⁻ cells (2985 TCR clonotypes) (A) or sorted PD-1⁺ cells (805 TCR clonotypes) (B) after 14 days of *in vitro* expansion.
Figure 7C is a graph showing the frequency (%) of unique TCR beta chain CDR3 region amino acid sequences of sorted PD-1⁻ cells (black circles) or sorted PD-1⁺ cells (grey circles).

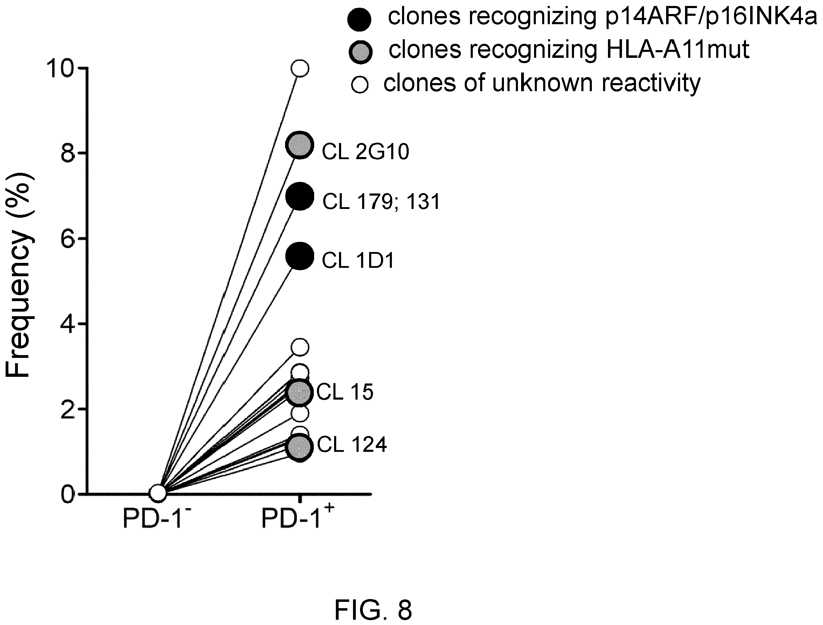

Figure 8 is a graph showing the frequency (%) of TCR β chain clonotypes in the PD-1⁻population or in the PD-1⁺ population that recognize mutated epitopes p14ARF/p16INK4a (black circles) or HLA-A11mut (grey circles) that are expressed specifically by the autologous tumor cell line and clonotypes with unknown reactivity (open circles).

### DETAILED DESCRIPTION OF THE INVENTION

It has been discovered that selecting CD8⁺ cells that also express any one or more of TIM-3 (T Cell Ig- and mucin-domain-containing molecule-3), LAG-3 (lymphocyte activation gene 3; CD223), 4-1BB (CD137), and PD-1 (CD279) biomarkers enriches for tumor-reactive T cells isolated from fresh tumor samples. Selecting the CD8⁺ cells that also express any one or more of PD-1, 4-1BB, TIM-3, and LAG-3 advantageously enriches for greater numbers of tumor-reactive T cells as compared to CD8⁺ cells that do not express these markers.

In this regard, an embodiment of the invention provides a method of obtaining a cell population enriched for tumor-reactive T cells, the method comprising: (a) obtaining a bulk population of T cells from a tumor sample; (b) specifically selecting CD8⁺ T cells that express any one or more of TIM-3, LAG-3, 4-1BB, and PD-1 from the bulk population; and (c) separating the cells selected in (b) from unselected cells to obtain a cell population enriched for tumor-reactive T cells. The inventive methods advantageously make it possible to shorten the time of *in vitro* culture of cells prior to administering the cells to a patient. Moreover, the inventive methods advantageously may provide a cell population enriched for tumor-reactive T cells that may be administered to a patient without having to screen for autologous tumor recognition.

The method may comprise obtaining a bulk population of T cells from a tumor sample by any suitable method known in the art. For example, a bulk population of T cells can be obtained from a tumor sample by dissociating the tumor sample into a cell suspension from which specific cell populations can be selected. Suitable methods of obtaining a bulk population of T cells may include, but are not limited to, any one or more of mechanically dissociating (e.g., mincing) the tumor, enzymatically dissociating (e.g., digesting) the tumor, and aspiration (e.g., as with a needle).

The bulk population of T cells obtained from a tumor sample may comprise any suitable type of T cell. Preferably, the bulk population of T cells obtained from a tumor sample comprises tumor infiltrating lymphocytes (TILs).

The tumor sample may be obtained from any mammal. Unless stated otherwise, as used herein, the term "mammal" refers to any mammal including, but not limited to, mammals of the order Logomorpha, such as rabbits; the order Carnivora, including Felines (cats) and Canines (dogs); the order Artiodactyla, including Bovines (cows) and Swines (pigs); or of the order Perssodactyla, including Equines (horses). It is preferred that the mammals are non-human primates, e.g., of the order Primates, Ceboids, or Simoids (monkeys) or of the order Anthropoids (humans and apes). In some embodiments, the mammal may be a mammal of the order Rodentia, such as mice and hamsters. Preferably, the mammal is a non-human primate or a human. An especially preferred mammal is the human.

The method may comprise specifically selecting CD8⁺ T cells that express any one or more of TIM-3, LAG-3, 4-1BB, and PD-1 from the bulk population. In a preferred embodiment, the method comprises selecting cells that also express CD3. The method may comprise specifically selecting the cells in any suitable manner. Preferably, the selecting is carried out using flow cytometry. The flow cytometry may be carried out using any suitable method known in the art. The flow cytometry may employ any suitable antibodies and stains. For example, the specific selection of CD3, CD8, TIM-3, LAG-3, 4-1BB, or PD-1 may be carried out using anti-CD3, anti-CD8, anti-TIM-3, anti-LAG-3, anti-4-1BB, or anti-PD-1 antibodies, respectively. Preferably, the antibody is chosen such that it specifically recognizes and binds to the particular biomarker being selected. The antibody or antibodies may be conjugated to a bead (e.g., a magnetic bead) or to a fluorochrome. Preferably, the flow cytometry is fluorescence-activated cell sorting (FACS).

In an embodiment of the invention, specifically selecting may comprise specifically selecting CD8⁺ T cells that are positive for expression of any one of TIM-3, LAG-3, 4-1BB, or PD-1, any combination of two or three of TIM-3, LAG-3, 4-1BB, and PD-1 or all four of TIM-3, LAG-3, 4-1BB, and PD-1. In this regard, specifically selecting may comprise specifically selecting T cells that are single positive for expression of any one of TIM-3, LAG-3, 4-1BB, and PD-1 or specifically selecting T cells that are double, triple, or quadruple positive for simultaneous co-expression of any two, three or four of TIM-3, LAG-3, 4-1BB, and PD-1. In an embodiment of the invention, the method comprises specifically selecting CD8⁺ T cells that express TIM-3 from the bulk population. In another embodiment, the method comprises specifically selecting CD8⁺ T cells that express LAG-3 from the bulk population. In still another embodiment, the method comprises specifically selecting CD8⁺ T cells that express 4-1BB from the bulk population. In still another embodiment of the invention, the method comprises specifically selecting CD8⁺ T cells that express PD-1 from the bulk population. An additional embodiment of the invention provides a method comprising specifically selecting CD8⁺ T cells that are (i) 4-1BB⁺/PD-1⁺, (ii) 4-1BB⁻/PD-1⁺, and/or (iii) 4-1BB⁺/PD-1⁻ from the bulk population. Another embodiment of the invention provides a method comprising specifically selecting CD8⁺ T cells that are (i) LAG-3⁺/PD-1⁺, (ii) LAG-3⁻/PD-1⁺, and/or (iii) LAG-3⁺/PD-1⁻ from the bulk population. Still another embodiment of the invention provides a method comprising specifically selecting CD8⁺ T cells that are (i) TIM-3⁺/PD-1⁺, (ii) TIM-3⁻/PD-1⁺, or (iii) TIM-3⁺/PD-1⁻from the bulk population. Still another embodiment of the invention provides a method comprising specifically selecting CD8⁺ T cells that are (i) TIM-3⁺/LAG-3⁺, (ii) TIM-3⁻/LAG-3⁺, or (iii) TIM-3⁺/LAG-3⁻ from the bulk population. Another embodiment of the invention provides a method comprising specifically selecting CD8⁺ T cells that are (i) 4-1BB⁺/LAG-3⁺, (ii) 4-1BB⁻/LAG-3⁺, or (iii) 4-1BB⁺/LAG-3⁻ from the bulk population. Still another embodiment of the invention provides a method comprising specifically selecting CD8⁺ T cells that are (i) 4-1BB⁺/TIM-3⁺, (ii) 4-1BB⁻/TIM-3⁺, or (iii) 4-1BB⁺/TIM-3⁻ from the bulk population. In another embodiment of the invention, any of the methods described herein may further comprise selecting cells that also express CD3⁺.

In an embodiment of the invention, specifically selecting may comprise specifically selecting combinations of CD8⁺ cells expressing any of the markers described herein. In this regard, the method may produce a cell population that is enriched for tumor-reactive cells that comprises a mixture of cells expressing any two, three, four, or more of the biomarkers described herein. In an embodiment of the invention, specifically selecting comprises specifically selecting any of the following combinations of cells: (a) PD-1⁺ cells and 4-1BB⁺ cells, (b) PD-1⁺ cells and LAG-3⁺ cells, (c) PD-1⁺ cells and TIM-3⁺ cells, (d) 4-1BB⁺ cells and LAG-3⁺ cells, (e) 4-1BB⁺ cells and TIM-3⁺ cells, (f) LAG-3⁺ cells and TIM-3⁺ cells, (g) PD-1⁺ cells, 4-1BB⁺ cells, and LAG-3⁺ cells, (h) PD-1⁺ cells, 4-1BB⁺ cells, and TIM-3⁺ cells, (i) PD-1⁺ cells, LAG-3⁺ cells, and TIM-3⁺ cells, (j) 4-1BB⁺ cells, LAG-3⁺ cells, and TIM-3⁺ cells, and/or (k) PD-1⁺ cells, 4-1BB⁺ cells, LAG-3⁺ cells, and TIM-3⁺ cells. In another embodiment of the invention, any of the methods described herein may further comprise selecting cells that also express CD8⁺ and/or CD3⁺.

The method may comprise separating the selected cells from unselected cells to obtain a cell population enriched for tumor-reactive T cells. In this regard, the selected cells may be physically separated from the unselected cells. The selected cells may be separated from unselected cells by any suitable method such as, for example, sorting. Separating the selected cells from the unselected cells preferably produces a cell population that is enriched for tumor-reactive T cells.

The cell populations obtained by the inventive methods are advantageously enriched for tumor-reactive T cells. In this regard, the cell populations obtained by the inventive methods may comprise a higher proportion of tumor reactive T cells as compared to cell populations that have not been obtained by sorting for expression of any one or more of TIM-3, LAG-3, 4-1BB, and PD-1.

In an embodiment of the invention, the method comprises obtaining the cell population enriched for tumor-reactive T cells without screening for autologous tumor recognition. In this regard, the inventive methods advantageously provide a cell population that is enriched for cells that have tumor reactivity without having to screen the cells for autologous tumor recognition.

In an embodiment of the invention, the method does not comprise non-specifically stimulating the bulk population of T cells prior to specifically selecting the cells. In this regard, the inventive methods advantageously provide a cell population that is enriched for tumor reactive T cells without stimulating the bulk population of T cells nonspecifically (e.g., with anti-4-1BB antibodies, anti-CD3 antibodies, anti-CD28 antibodies).

In an embodiment of the invention, the method further comprises expanding the numbers of T cells in the enriched cell population obtained by the inventive methods *in vitro.* The numbers of T cells may be increased at least about 3-fold (or 4-, 5-, 6-, 7-, 8-, or 9-fold), more preferably at least about 10-fold (or 20-, 30-, 40-, 50-, 60-, 70-, 80-, or 90-fold), more preferably at least about 100-fold, more preferably at least about 1,000 fold, or most preferably at least about 100,000-fold. The numbers of T cells may be expanded using any suitable method known in the art. Exemplary methods of expanding the numbers of cells are described in U.S. Patent 8,034,334 and U.S. Patent Application Publication No. 2012/0244133, each of which is incorporated herein by reference.

In an embodiment of the invention, the method further comprises culturing the enriched cell population obtained by the inventive methods in the presence of any one or more of TWS119, interleukin (IL)-21, IL-12, IL-15, IL-7, transforming growth factor (TGF) beta, and AKT inhibitor (AKTi). Without being bound to a particular theory, it is believed that culturing the enriched cell population in the presence of TWS119, IL-21, and/or IL-12 may, advantageously, enhance the anti-tumor reactivity of the enriched cell population by preventing or retarding the differentiation of the enriched cell population.

In an embodiment of the invention, the method further comprises transducing or transfecting the cells of the enriched population obtained by any of the inventive methods described herein with a nucleotide sequence encoding any one or more of IL-12, IL-7, IL-15, IL-2, IL-21, mir155, and anti-PD-1 siRNA.

In an embodiment of the invention, the method further comprises stimulating the enriched cell population obtained by the inventive methods with a cancer antigen and/or with autologous tumor cells. Stimulating the enriched cell population with a cancer antigen and/or with autologous tumor cells may be carried out by any suitable method. For example, stimulating the enriched cell population may be carried out by physically contacting the enriched cell population with a cancer antigen and/or with autologous tumor cells. Without being bound to a particular theory, it is believed that stimulating the enriched cell population with a cancer antigen and/or with autologous tumor cells may, advantageously, enhance the anti-tumor reactivity of the enriched cell population.

The term "cancer antigen" as used herein refers to any molecule (e.g., protein, peptide, lipid, carbohydrate, etc.) solely or predominantly expressed or over-expressed by a tumor cell or cancer cell, such that the antigen is associated with the tumor or cancer. The cancer antigen can additionally be expressed by normal, non-tumor, or non-cancerous cells. However, in such cases, the expression of the cancer antigen by normal, non-tumor, or non-cancerous cells is not as robust as the expression by tumor or cancer cells. In this regard, the tumor or cancer cells can over-express the antigen or express the antigen at a significantly higher level, as compared to the expression of the antigen by normal, non-tumor, or non-cancerous cells. Also, the cancer antigen can additionally be expressed by cells of a different state of development or maturation. For instance, the cancer antigen can be additionally expressed by cells of the embryonic or fetal stage, which cells are not normally found in an adult host. Alternatively, the cancer antigen can be additionally expressed by stem cells or precursor cells, which cells are not normally found in an adult host.

The cancer antigen can be an antigen expressed by any cell of any cancer or tumor, including the cancers and tumors described herein. The cancer antigen may be a cancer antigen of only one type of cancer or tumor, such that the cancer antigen is associated with or characteristic of only one type of cancer or tumor. Alternatively, the cancer antigen may be a cancer antigen (e.g., may be characteristic) of more than one type of cancer or tumor. For example, the cancer antigen may be expressed by both breast and prostate cancer cells and not expressed at all by normal, non-tumor, or non-cancer cells. Exemplary cancer antigens may include any one or more of gp100, MART-1, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, NY-ESO-1, vascular endothelial growth factor receptor-2 (VEGFR-2), HER-2, mesothelin, and epidermal growth factor receptor variant III (EGFR III).

The inventive methods advantageously produce cell populations enriched for tumor-reactive T cells. The T cells may be tumor-reactive such that they specifically recognize, lyse, and/or kill tumor cells. In this regard, an embodiment of the invention provides an isolated or purified cell population enriched for tumor-reactive T cells obtained by any of the inventive methods described herein. In an embodiment, the isolated or purified cell population comprises any one or more of (a) CD8⁺/4-1BB⁺/PD-1⁺ T cells, (b) CD8⁺/4-1BB-/PD-1⁺ T cells, (c) CD8⁺/4-1BB⁺/PD-1T cells, (d) CDS⁺/LAG-3⁺/PD-1⁺ T cells, (e) CD8⁺ALAG-3⁻/PD-1⁺ T cells, (f) CD8⁺/LAG-3⁺/PD-1⁻ T cells, (g) CD8⁺/TIM-3⁺/PD-1⁺ T cells, (h) CD8⁺/TIM-3⁻/PD-1⁺ T cells, (i) CD8⁺/TIM-3⁺/PD-1⁻ T cells, (j) CD8⁺/TIM-3⁺/LAG-3⁺ T cells, (k) CD8⁺/TIM-3⁻ALAG-3⁺ T cells, (1) CD8⁺/TIM-3⁺/LAG-3⁻ T cells, (m) CD8⁺/4-1BB⁺/LAG-3⁺ T cells, (n) CD8⁺/4-1BB⁻/LAG-3⁺ T cells, (o) CD8⁺/4-1BB⁺/LAG-3⁻ T cells, (p) CD8⁺/4-1BB⁺/TIM-3⁺ T cells, (q) CD8⁺/4-1BB⁻/TIM-3⁺ T cells, and (r) CD8⁺/4-1BB⁺/TIM-3⁻ T cells, wherein the cell population is enriched for tumor-reactive T cells. In another embodiment of the invention, the isolated or purified cell population comprises (a) CD8⁺/4-1BB⁺/PD-1⁺ T cells, (b) CD8⁺/4-1BB-/PD-1⁺ T cells, (c) CD8⁺/4-1BB⁺/PD-1T cells, (d) CD8⁺/LAG-3⁺/PD-1⁺ T cells, (e) CD8⁺/LAG-3/PD-1⁺ T cells, (f) CD8⁺/LAG-3⁺/PD-1⁻ T cells, (g) CD8⁺/TIM-3⁺/PD-1⁺ T cells, (h) CD8⁺/TIM-3⁻/PD-1⁺ T cells, (i) CD8⁺/TIM-3⁺/PD-1⁻ T cells, (j) CD8⁺/TIM-3⁺/LAG-3⁺ T cells, (k) CD8⁺/TIM-3⁻/LAG-3⁺ T cells, (1) CD8⁺/TIM-3⁺/LAG-3⁻ T cells, (m) CD8⁺/4-1BB⁺/LAG-3⁺ T cells, (n) CD8⁺/4-1BB/LAG-3⁺ T cells, (o) CD8⁺/4-1BB⁺/LAG-3⁻ T cells, (p) CD8⁺/4-1BB⁺/TIM-3⁺ T cells, (q) CD8⁺/4-1BB⁻/TIM-3⁺ T cells, or (r) CD8⁺/4-1BB⁺/TIM-3⁻ T cells. In another embodiment of the invention, any of the cell populations described herein may also be CD3⁺.

In an embodiment of the invention, the isolated or purified cell population comprises a mixture of cells expressing any of the biomarkers described herein. For example, the isolated or purified cell population may comprise a combination of (a) PD-1⁺ cells and 4-1BB⁺ cells, (b) PD-1⁺ cells and LAG-3⁺ cells, (c) PD-1⁺ cells and TIM-3⁺ cells, (d) 4-1BB⁺ cells and LAG-3⁺ cells, (e) 4-1BB⁺ cells and TIM-3⁺ cells, (f) LAG-3⁺ cells and TIM-3⁺ cells, (g) PD-1⁺ cells, 4-1BB⁺ cells, and LAG-3⁺ cells, (h) PD-1⁺ cells, 4-1BB⁺ cells, and TIM-3⁺ cells, (i) PD-1⁺ cells, LAG-3⁺ cells, and TIM-3⁺ cells, (j) 4-1BB⁺ cells, LAG-3⁺ cells, and TIM-3⁺ cells, and/or (k) PD-1⁺ cells, 4-1BB⁺ cells, LAG-3⁺ cells, and TIM-3⁺ cells. In another embodiment of the invention, any of the cell populations described herein may also be CD8⁺ and/or CD3⁺.

The term "isolated" as used herein means having been removed from its natural environment. The term "purified" as used herein means having been increased in purity, wherein "purity" is a relative term, and not to be necessarily construed as absolute purity. For example, the purity can be at least about 50%, can be greater than 60%, 70% or 80%, 90% or can be 100%.

Another embodiment of the invention provides a method of administering a cell population enriched for tumor-reactive T cells to a mammal, the method comprising: (a) obtaining a bulk population of T cells from a tumor sample; (b) specifically selecting CD8⁺ T cells that express any one or more of TIM-3, LAG-3, 4-1BB, and PD-1 from the bulk population; (c) separating the cells selected in (b) from unselected cells to obtain a cell population enriched for tumor-reactive T cells; and (d) administering the cell population enriched for tumor-reactive T cells to the mammal. Obtaining a bulk population of T cells from a tumor sample, specifically selecting CD8⁺ T cells that express any one or more of TIM-3, LAG-3, 4-1BB, and PD-1 from the bulk population, and separating the selected cells from unselected cells to obtain a cell population may be carried out as described herein with respect to other aspects of the invention.

The method may further comprise administering the cell population enriched for tumor-reactive T cells to the mammal. The cell population enriched for tumor-reactive T cells may be administered in any suitable manner. Preferably, the cell population enriched for tumor-reactive T cells is administered by injection, e.g., intravenously.

The inventive cell population enriched for tumor-reactive T cells can be included in a composition, such as a pharmaceutical composition. In this regard, the invention provides a pharmaceutical composition comprising any of the cell populations described herein and a pharmaceutically acceptable carrier.

Another embodiment of the invention provides a method of obtaining a pharmaceutical composition comprising a cell population enriched for tumor-reactive T cells, the method comprising: (a) obtaining a bulk population of T cells from a tumor sample; (b) specifically selecting CD8⁺ T cells that express any one or more of TIM-3, LAG-3, 4-1BB, and PD-1 from the bulk population; (c) separating the cells selected in (b) from unselected cells to obtain a cell population enriched for tumor-reactive T cells; and (d) combining the cell population enriched for tumor-reactive T cells with a pharmaceutically acceptable carrier to obtain a pharmaceutical composition comprising a cell population enriched for tumor-reactive T cells. Obtaining a bulk population of T cells from a tumor sample, specifically selecting CD8⁺ T cells that express any one or more of TIM-3, LAG-3, 4-1BB, and PD-1 from the bulk population, and separating the selected cells from unselected cells to obtain a cell population may be carried out as described herein with respect to other aspects of the invention.

The method may comprise combining the cell population enriched for tumor-reactive T cells with a pharmaceutically acceptable carrier to obtain a pharmaceutical composition comprising a cell population enriched for tumor-reactive T cells. Preferably, the carrier is a pharmaceutically acceptable carrier. With respect to pharmaceutical compositions, the carrier can be any of those conventionally used for the administration of cells. Such pharmaceutically acceptable carriers are well-known to those skilled in the art and are readily available to the public. It is preferred that the pharmaceutically acceptable carrier be one which has no detrimental side effects or toxicity under the conditions of use. A suitable pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment, the pharmaceutically acceptable carrier is supplemented with human serum albumen.

For purposes of the invention, the dose, e.g., number of cells in the inventive cell population enriched for tumor-reactive T cells, administered should be sufficient to effect, e.g., a therapeutic or prophylactic response, in the mammal over a reasonable time frame. For example, the number of cells should be sufficient to bind to a cancer antigen, or detect, treat or prevent cancer in a period of from about 2 hours or longer, e.g., 12 to 24 or more hours, from the time of administration. In certain embodiments, the time period could be even longer. The number of cells will be determined by, e.g., the efficacy of the particular cells and the condition of the mammal (e.g., human), as well as the body weight of the mammal (e.g., human) to be treated.

Many assays for determining an administered number of cells from the inventive cell population enriched for tumor-reactive T cells are known in the art. For purposes of the invention, an assay, which comprises comparing the extent to which target cells are lysed or one or more cytokines such as, e.g., IFN-γ and IL-2 are secreted upon administration of a given number of such cells to a mammal among a set of mammals of which is each given a different number of the cells, could be used to determine a starting number to be administered to a mammal. The extent to which target cells are lysed, or cytokines such as, e.g., IFN-γ and IL-2 are secreted, upon administration of a certain number of cells, can be assayed by methods known in the art. Secretion of cytokines such as, e.g., IL-2, may also provide an indication of the quality (e.g., phenotype and/or effectiveness) of a cell preparation.

The number of the cells from the inventive cell population enriched for tumor-reactive T cells also will be determined by the existence, nature and extent of any adverse side effects that might accompany the administration of a particular cell population. Typically, the attending physician will decide the number of the cells with which to treat each individual patient, taking into consideration a variety of factors, such as age, body weight, general health, diet, sex, route of administration, and the severity of the condition being treated. By way of example and not intending to limit the invention, the number of cells can be about 10 x 10⁶ to about 10 x 10¹¹ cells per infusion, about 10 x 10⁹ cells to about 10 x 10¹¹ cells per infusion, or 10 x 10⁷ to about 10 x 10⁹ cells per infusion. The cell populations obtained by the inventive methods may, advantageously, make it possible to effectively treat or prevent cancer.

It is contemplated that the cell populations obtained by the inventive methods can be used in methods of treating or preventing cancer. In this regard, the invention provides a method of treating or preventing cancer in a mammal, comprising administering to the mammal the pharmaceutical compositions or cell populations obtained by any of the inventive methods described herein in an amount effective to treat or prevent cancer in the mammal. Another embodiment of the invention provides a method of treating or preventing cancer in a mammal, comprising administering a cell population enriched for tumor-reactive T cells to a mammal by any of the inventive methods described herein in an amount effective to treat or prevent cancer in the mammal.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount or any level of treatment or prevention of cancer in a mammal. Furthermore, the treatment or prevention provided by the inventive method can include treatment or prevention of one or more conditions or symptoms of the disease, e.g., cancer, being treated or prevented. Also, for purposes herein, "prevention" can encompass delaying the onset of the disease, or a symptom or condition thereof.

For purposes of the inventive methods, wherein populations of cells are administered, the cells can be cells that are allogeneic or autologous to the mammal. Preferably, the cells are autologous to the mammal.

An embodiment of the invention further comprises lymphodepleting the mammal prior to administering any of the enriched cell populations obtained by any of the inventive methods described herein. Examples of lymphodepletion include, but may not be limited to, nonmyeloablative lymphodepleting chemotherapy, myeloablative lymphodepleting chemotherapy, total body irradiation, etc.

With respect to the inventive methods, the cancer can be any cancer, including any of sarcomas (e.g., synovial sarcoma, osteogenic sarcoma, leiomyosarcoma uteri, and alveolar rhabdomyosarcoma), lymphomas (e.g., Hodgkin lymphoma and non-Hodgkin lymphoma), hepatocellular carcinoma, glioma, head-neck cancer, acute lymphocytic cancer, acute myeloid leukemia, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vulva, chronic lymphocytic leukemia, chronic myeloid cancer, colon cancer (e.g., colon carcinoma), esophageal cancer, cervical cancer, gastrointestinal cancer (e.g., gastrointestinal carcinoid tumor), hypopharynx cancer, larynx cancer, liver cancer, lung cancer, malignant mesothelioma, melanoma, multiple myeloma, nasopharynx cancer, ovarian cancer, pancreatic cancer, peritoneum, omentum, and mesentery cancer, pharynx cancer, prostate cancer, rectal cancer, renal cancer, small intestine cancer, soft tissue cancer, stomach cancer, testicular cancer, thyroid cancer, ureter cancer, and urinary bladder cancer.

The following examples further illustrate the invention but, of course, should not be construed as in any way limiting its scope.

### EXAMPLE 1

This example demonstrates the frequency of CD3⁺/CD8⁺ cells in a fresh melanoma tumor digest sample expressing PD-1, TIM-3, LAG-3 or 4-1BB. This example also demonstrates that co-expression of 1) TIM-3 and PD-1, 2) LAG-3 and PD-1, and 3) LAG-3 and TIM-3 by CD8⁺ T cells isolated from a fresh melanoma tumor sample. This example also demonstrates the expression of PD-1, TIM-3, or LAG-3 by MART-1₂₇₋₃₅ reactive cells.

Single cell suspensions obtained from a mechanical and enzymatic digest of a fresh melanoma tumor sample were thawed and rested overnight at 1 x 10⁶ cells/ml in absence of cytokines. The cells were stained and the percentage of CD3⁺CD8⁺ cells expressing PD-1, TIM-3, LAG-3, 4-1BB, OX40, CD25, CD28, CD27, or CD70 was measured by flow cytometry. The results are shown in Figure 1A. As shown in Figure 1A, CD3⁺/CD8⁺ cells from a fresh tumor digest sample can express PD-1, TIM-3, LAG-3 or 4-1BB.

In a separate experiment, cells were obtained from fresh samples of two different melanoma tumors and the co-expression of TIM-3 and PD-1, the co-expression of LAG-3 and PD-1, and the co-expression of LAG-3 and TIM-3 was measured using flow cytometry gated on live cells and CD3⁺ CD8⁺ cells. The results showed that subsets of CD8⁺ T cells infiltrating melanoma tumors co-express 1) TIM-3 and PD-1, 2) LAG-3 and PD-1, and 3) LAG-3 and TIM-3.

In a separate experiment, the expression of PD-1, TIM-3, or LAG-3 on MART-1₂₇-₃₅ reactive T cells was measured using flow cytometry gated on live cells and CD3⁺ CD8⁺ cells and compared to that of CD3⁺ CD8⁺ T cells that were not MART-1₂₇₋₃₅ reactive. The results showed that MART-1₂₇₋₃₅ reactive cells infiltrating melanoma tumors express higher levels of PD-1, TIM-3, and LAG-3 as compared CD3⁺ CD8⁺ T cells that were not MART-1₂₇₋₃₅ reactive.

### EXAMPLE 2

This example demonstrates a method of specifically selecting CD3⁺ CD8⁺ cells that also express one of PD-1, TIM-3, LAG-3 and 4-1BB and expanding the numbers of the selected cells.

A single cell suspension obtained from a fresh melanoma tumor sample (FrTu#1913) was thawed and rested overnight in absence of cytokines and then stained. The cells were sorted into the following CD3⁺ populations using anti-CD3, anti-CD8, anti-PD-1, TIM-3, LAG-3 and 4-1BB antibodies: CD8⁺, CD8⁺/PD-1⁺, CD8⁺/LAG3⁺, CD8⁺/TIM-3⁺, CDS⁺/4-1BB⁺, CD8⁺/PD-1⁻, CD8⁺/LAG3⁻, CD8⁺/TIM-3⁻, or CD8⁺/4-1BB⁻ by fluorescence-activated cell sorting (FACS). The numbers of cells were then expanded using a rapid expansion protocol (200-fold excess irradiated feeders, 30 ng/ml anti-CD3 and 500 CU/ml IL-2) and fold-expansion of the isolated populations was measured. The results are shown in Figure 1B. As shown in Figure 1B, the numbers of CD8⁺ cells that also express one of PD-1, TIM-3, LAG-3 and 4-1BB were expanded.

### EXAMPLE 3

This example demonstrates the *in vitro* reactivity of T cells isolated from a fresh melanoma tumor sample and sorted for expression of CD8 and one of PD-1, LAG-3, TIM-3, and 4-1BB.

4-1BB up-regulation is an indicator of TCR stimulation. It has been observed that after the numbers of T cells are expanded and in the absence of TCR stimulation, 4-1BB expression is lost. It has also been observed that after the numbers of cells are expanded and the cells are co-cultured with an autologous tumor cell line, T cells that had previously lost 4-1BB expression and which are stimulated by the tumor cell line will re-express 4-1BB. Accordingly, 4-1BB expression is measured 24 hours after co-culture with autologous tumor as a marker of TCR stimulation against the autologous tumor cell line.

A single cell suspension from a fresh melanoma tumor digest sample (FrTu#1913) was rested overnight without cytokines and sorted for the following populations: CD8⁺, CD8⁺/PD-1⁺, CD8⁺/LAG3⁺, CD8⁺/TIM-3⁺, CD8⁺/4-1BB⁺, CD8⁺/PD-1⁻, CD8⁺/LAG3⁻, CD8⁺/TIM-3⁻, or CD8⁺/4-1BB⁻ populations by FACS as described in Example 3. The numbers of sorted cells were expanded *in vitro* for 14 days. On day 14, the cells were washed and co-cultured against an autologous tumor cell line (1 x 10⁵ effectors: 1 x 10⁵ target cells). Reactivity was assessed by quantifying IFN-gamma release and the percentage of CD8⁺ cells expressing 4-1BB 24 hours after co-culture with an autologous tumor cell line (TC1913) and allogeneic (Allo.) tumor cell lines. The percentage of CD8⁺ cells recognizing a specific mutated epitope (CDKn2A) targeted by T cells was also quantified using a tetramer against this particular epitope. The results are shown in Tables 1 and 2 and in Figures 2A-2E.

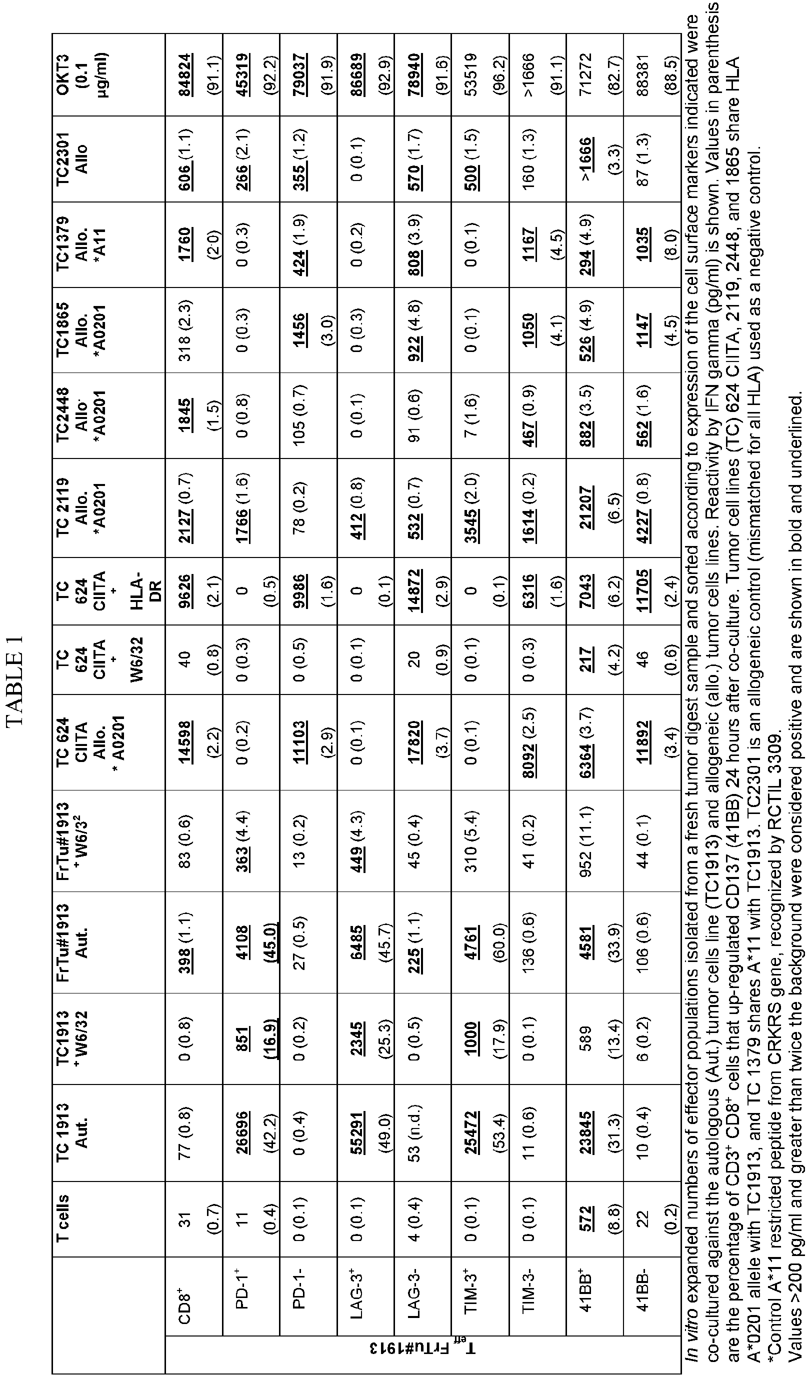

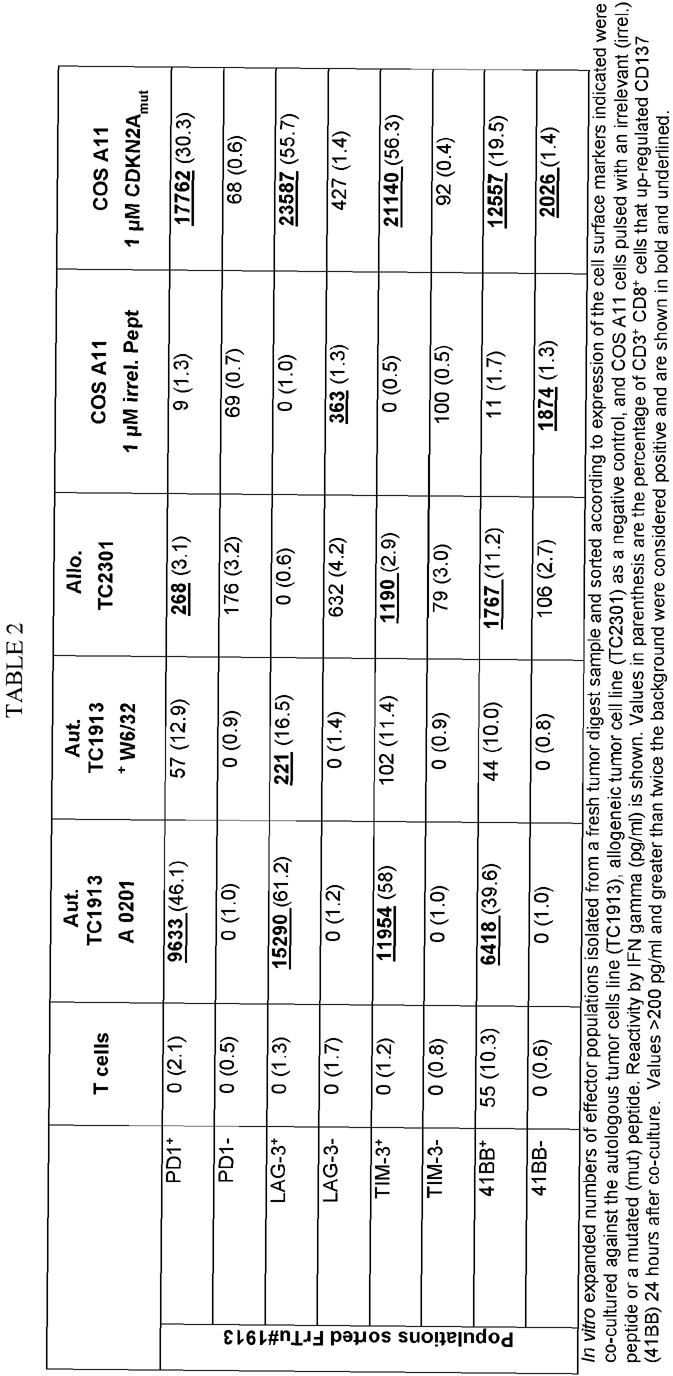

As shown in Tables 1 and 2, T cells isolated from a fresh melanoma tumor sample and sorted for expression of CD8 and one of PD-1, LAG-3, TIM-3, and 4-1BB have reactivity against autologous tumor cell lines as measured by IFN-gamma secretion, 4-1BB expression, and percentage of cells recognizing CDKn2A. As shown in Figures 2A-2E, T cells isolated from each of five different fresh melanoma tumor samples and sorted for expression of CD8 and one of PD-1, LAG-3, TIM-3, and 4-1BB have reactivity against autologous tumor cell lines as measured by IFN-gamma secretion and 4-1BB expression.

In a separate experiment, cells were isolated from two independent fresh melanoma tumor samples (FrTu#1913 and FrTu#3713) and sorted for expression of CD8 and for expression of PD-1, LAG-3, TIM-3 or 4-1BB as described in Example 3. The numbers of the sorted cells were expanded for 14 days *in vitro.* On day 15, target tumor cell lines (autologous and allogeneic) were labeled with ⁵¹Cr and co-cultured for 4 hours with effector cells at the ratios shown in Figures 3A-3F. ⁵¹Cr release was determined in triplicate by gamma-counting and the percentage of specific lysis was calculated using the following formula: [(experimental counts per minute (cpm) - spontaneous cpm)/(maximal cpm - spontaneous cpm)] × 100. The results are shown in Figures 3A-3F. As shown in Figures 3A-3F, cells sorted for expression of PD-1, LAG-3, TIM-3 or 4-1BB were capable of lysing at autologous tumor cell lines.

### EXAMPLE 4

This example demonstrates the reactivity of CD8⁺ cells isolated from a melanoma tumor sample and sorted for expression of 4-1BB and/or PD-1.

Cells were isolated from fresh melanoma tumor samples from 3 patients and were sorted for CD3⁺/CD8⁺/4-1BB⁺/PD-1⁻, CD3⁺/CD8⁺/4-1BB⁺/PD-1⁺, CD3⁺/CD8⁺/4-1BB/PD-1⁺, CD3⁺/CD8⁺/4-1BB-/PD-1⁻, CD3⁺/CD8⁺/PD-1⁺, CD3⁺/CD8⁺/4-1BB⁺, CD3⁺/CD8⁺/PD-1⁻, or CD3⁺/CDS⁺/4-1BB⁻ populations by FACS. Sorted cells were co-cultured with autologous tumor cells, and up-regulation of 4-1BB expression was measured by flow cytometry. For all three tumor samples, the results showed that T cells recognizing autologous tumor (as measured by up-regulation of 4-1BB expression) can be found in single positive PD-1⁺ or 4-1BB⁺ expressing cells, but the highest frequency of tumor-reactive cells (as measured by 4-1BB up-regulation) was found in the population co-expressing both 4-1BB and PD-lin the fresh melanoma tumor digest sample.

In a separate experiment, cells were isolated from a fresh melanoma tumor sample (FrTu#1913), were sorted for CD3⁺/CD8⁺/4-1BB⁺/PD-1⁺, CD3⁺/CD8⁺/4-1BB/PD-1⁺, and CD3⁺/CD8⁺/4-1BB⁻/PD-1⁻ populations by FACS, and clones were established from the sorted cells. The clones were co-cultured with autologous tumor cell lines, and up-regulation of 4-1BB expression was measured by flow cytometry and IFN-gamma secretion was measured. The results showed that the highest frequency of tumor-reactive clones (as measured by 4-1BB up-regulation and IFN-gamma secretion) was found in the population co-expressing both PD-1 and 4-1BB.

In a separate experiment, a single cell suspension from melanoma tumor FrTu#3713 was rested overnight without cytokines and the cells were sorted for CD8⁺, CD8⁺/PD-1⁺, CD8⁺PD-1⁻, CD8⁺/4-1BB⁺, CD8⁺/4-1BB⁻, CD8⁺/4-1BB⁺/PD-1⁻, CD8⁺/4-1BB⁺/PD-1⁺, CD8⁺/4-1BB /PD-1⁺, and CD8⁺/4-1BB⁻/PD-1⁻ populations by FACS. A single cell suspension from melanoma tumor FrTu#3612 was rested overnight without cytokines and the cells were sorted for CD8⁺, CD8⁺/PD-1⁺, CD8⁺PD-1⁻, CD8⁺/4-1BB⁺/PD-1⁻, CD8⁺/4-1BB⁺/PD-1⁺, CD8⁺/4-1BB /PD-1⁺, and CD8⁺/4-1BB⁻/PD-1⁻ populations by FACS. The numbers of sorted cells were expanded for 14 days *in vitro.* On day 14, the cells were washed and co-cultured against the autologous tumor cell line (1 x 10⁵ effectors: 1 x 10⁵ target cells) and reactivity was assessed by quantifying the percentage of CD8⁺ cells expressing 4-1BB (FrTu#3612 and FrTu#3713) and/or the amount of IFN-gamma secretion (FrTu#3612) 24 hours after co-culture. The results are shown in Figures 4A and 4B. As shown in Figure 4A, the cells sorted for double-positive PD-1 and 4-1BB co-expression displayed similar levels of 4-1BB up-regulation as that demonstrated by cells sorted based on single positive PD-1 or 4-1BB expression. As shown in Figure 4B, the cells sorted for double-positive PD-1 and 4-1BB co-expression displayed similar levels of 4-1BB up-regulation and IFN-gamma secretion as that demonstrated by cells sorted based on single positive PD-1 expression.

In a separate experiment, cells isolated from melanoma tumor FrTu#3713 were sorted for CD8⁺/4-1BB⁺/PD-1⁻, CD8⁺/4-1BB⁺/PD-1⁺, CD8⁺/4-1BB⁻/PD-1⁺, and CD8⁺/4-1BB⁻/PD-1⁻ populations by FACS. The numbers of sorted cells were expanded for 14 days *in vitro.* On day 15, target tumor cell lines (autologous and allogeneic) were labeled with ⁵¹Cr and co-cultured for 4 hours with effector cells at the ratios indicated in Figures 5A-5C. ⁵¹Cr release was determined in triplicate by γ-counting and the percentage of specific lysis was calculated using the following formula: [(experimental cpm - spontaneous cpm)/(maximal cpm - spontaneous cpm)] × 100. The results are shown in Figures 5A-5C. As shown in Figures 5A-5C, cells sorted for 4-1BB⁺ single positive expression, PD-1⁺ single positive expression, or double positive 4-1BB⁺/PD-1⁺ expression are capable of lysing the autologous tumor cells *in vitro.*

### EXAMPLE 5

This example demonstrates the reactivity of CD8⁺ cells isolated from a gastrointestinal (GI) tract tumor sample and sorted for expression of PD-1, TIM-3, or 4-1BB.

A single cell suspension from a fresh gastrointestinal (GI) tract tumor sample (FrTu#3446b) was rested overnight without cytokines and sorted according to expression of PD-1, TIM-3, or 4-1BB by FACS. The numbers of sorted cells were expanded *in vitro* for 14 days. On day 14, cells were washed and co-cultured against the autologous tumor cell line (1 x 10⁵ effectors: 1 x 10⁵ target cells) and reactivity was assessed by quantifying IFN-gamma release and the percentage of CD8⁺ cells expressing 4-1BB 24 hours after co-culture. The results are shown in Figure 6. As shown in Figure 6, cells that were sorted according to PD-1, TIM-3, or 4-1BB expression demonstrated greater tumor reactivity as measured by 4-1BB expression as compared to those cell populations that lacked PD-1, TIM-3, or 4-1BB expression, respectively. Although no IFN-gamma secretion was detected, the specific up-regulation of 4-1BB indicates that the cells were tumor-reactive.

### EXAMPLE 6

This example demonstrates that PD-1⁺ sorted cells are more oligoclonal than PD-1⁻ cells after the numbers of cells are expanded *in vitro.* This example also demonstrates that PD-1⁺ sorted cells include clones targeting mutated epitopes expressed by autologous tumor after the numbers of cells are expanded *in vitro.*

A single cell suspension from a fresh melanoma tumor digest sample (FrTu#1913) was rested overnight without cytokines and sorted according to expression of PD-1 by FACS. The numbers of sorted cells were expanded *in vitro* for 14 days. TCR beta chain RNA was extracted using a µMACS RNA isolation kit (Miltenyi Biotec, Auburn, CA). cDNA synthesis and 5' RACE was carried out. Bar codes were introduced to the ends of the PCR product by PCR for identification of samples. The PCR product was washed and the library size was quantified. Deep sequencing was carried out (Illumina, Inc., San Diego, CA). The frequency of each unique TCR beta chain CDR3 region amino acid sequence in the population was determined. The results are shown in Figures 7A-7C. As shown in Figures 7A-7C, the PD-1⁺ sorted cells are more oligoclonal than PD-1⁻ cells after the numbers of cells are expanded *in vitro.*

The 20 most frequent clonotypes in the PD-1⁺ population are shown in Figure 8. As shown in Figure 8, the most frequent TCR beta chain clonotypes in PD-1⁺ sorted cells after numbers of cells were expanded were found at a low frequency in the PD-1⁻ fraction. As shown in Figure 8, clones recognizing mutated epitopes that are expressed by autologous tumor cell line were found within the 20 most frequent clones in the PD-1⁺ population and at a very low frequency in the PD-1⁻ population. These results demonstrate that tumor-reactive clones targeting mutated epitopes initially expressed PD-1 in the fresh tumor sample.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Further embodiments of the invention are described herein:
1. A method of obtaining a cell population enriched for tumor-reactive T-cells, the method comprising:
   (a) obtaining a bulk population of T cells from a tumor sample;
   (b) specifically selecting CD8⁺ T cells that express any one or more of TIM-3, LAG-3, 4-1BB, and PD-1 from the bulk population; and
   (c) separating the cells selected in (b) from unselected cells to obtain a cell population enriched for tumor-reactive T cells.
2. A method of obtaining a pharmaceutical composition comprising a cell population enriched for tumor-reactive T cells, the method comprising:
   (a) obtaining a bulk population of T cells from a tumor sample;
   (b) specifically selecting CD8⁺ T cells that express any one or more of TIM-3, LAG-3, 4-1BB, and PD-1 from the bulk population;
   (c) separating the cells selected in (b) from unselected cells to obtain a cell population enriched for tumor-reactive T cells; and
   (d) combining the cell population enriched for tumor-reactive T cells with a pharmaceutically acceptable carrier to obtain a pharmaceutical composition comprising a cell population enriched for tumor-reactive T cells.
3. The method of embodiment 1 or 2, wherein (b) comprises specifically selecting CD8⁺ T cells that express TIM-3 from the bulk population.
4. The method of any one of embodiments 1-3, wherein (b) comprises specifically selecting CD8⁺ T cells that express LAG-3 from the bulk population.
5. The method of any one of embodiments 1-4, wherein (b) comprises specifically selecting CD8⁺ T cells that express 4-1BB from the bulk population.
6. The method of any one of embodiments 1-5, wherein (b) comprises specifically selecting CD8⁺ T cells that express PD-1 from the bulk population.
7. The method of embodiment 1 or 2, wherein (b) comprises specifically selecting CD8⁺ T cells that are (i) 4-1BB⁺/PD-1⁺, (ii) 4-1BB⁻/PD-1⁺, and/or (iii) 4-1BB⁺/PD-1⁻ from the bulk population.
8. The method of embodiment 1 or 2, wherein (b) comprises specifically selecting CD8⁺ T cells that are (i) LAG-3⁺/PD-1⁺, (ii) LAG-3⁻/PD-1⁺, and/or (iii) LAG-3⁺/PD-1⁻ from the bulk population.
9. The method of embodiment 1 or 2, wherein (b) comprises specifically selecting CD8⁺ T cells that are (i) TIM-3⁺/PD-1⁺, (ii) TIM-3/PD-1⁺, or (iii) TIM-3⁺/PD-1⁻ from the bulk population.
10. The method of embodiment 1 or 2, wherein (b) comprises specifically selecting CD8⁺ T cells that are (i) TIM-3⁺/LAG-3⁺, (ii) TIM-3⁻/LAG-3⁺, or (iii) TIM-3⁺/LAG-3⁻ from the bulk population.
11. The method of embodiment 1 or 2, wherein (b) comprises specifically selecting CD8⁺ T cells that are (i) 4-1BB⁺/LAG-3⁺, (ii) 4-1BB⁻/LAG-3⁺, or (iii) 4-1BB⁺/LAG-3⁻ from the bulk population.
12. The method of embodiment 1 or 2, wherein (b) comprises specifically selecting CD8⁺ T cells that are (i) 4-1BB⁺/TIM-3⁺, (ii) 4-1BB⁻/TIM-3⁺, or (iii) 4-1BB⁺/TIM-3⁻ from the bulk population.
13. The method of any one of embodiments 1-12, wherein the cell population enriched for tumor-reactive T cells is obtained without screening for autologous tumor recognition.
14. The method of any one of embodiments 1-13, wherein the bulk population of T cells is not non-specifically stimulated prior to (b).
15. The method of any one of embodiments 1-14, further comprising expanding the numbers of T cells in the enriched cell population obtained in (c).
16. The method of any one of embodiments 1-15, further comprising culturing the enriched cell population obtained in (c) in the presence of any one or more of TWS119, interleukin (IL-21), IL-12, IL-15, IL-7, transforming growth factor (TGF) beta, and AKT inhibitor (AKTi).
17. The method of any one of embodiments 1-16, further comprising stimulating the enriched cell population obtained in (c) with a cancer antigen and/or with autologous tumor cells.
18. The method of any one of embodiments 1-17, further comprising transducing or transfecting the cells of the enriched population obtained in (c) with a nucleotide sequence encoding any one or more of IL-12, IL-7, IL-15, IL-2, IL-21, mir155, and anti-PD-1 siRNA.
19. An isolated or purified cell population enriched for tumor-reactive T cells obtained by the method of any one of embodiments 1-18.
20. An isolated or purified cell population comprising any one or more of:
   (a) CD8⁺/4-1BB⁺/PD-1⁺ T cells,
   (b) CD8⁺/4-1BB⁻/PD-1⁺ T cells,
   (c) CD8⁺/4-1BB⁺/PD-1⁻ T cells,
   (d) CD8⁺/LAG-3⁺/PD-1⁺ T cells,
   (e) CD8⁺/LAG-3⁻/PD-1⁺ T cells,
   (f) CD8⁺/LAG-3⁺/PD-1⁻ T cells,
   (g) CD8⁺/TIM-3⁺/PD-1⁺ T cells,
   (h) CD8⁺/TIM-3⁻/PD-1⁺ T cells,
   (i) CD8⁺/TIM-3⁺/PD-1⁻ T cells,
   (j) CD8⁺/TIM-3⁺/LAG-3⁺ T cells,
   (k) CD8⁺/TIM-3⁻/LAG-3⁺ T cells,
   (l) CD8⁺/TIM-3⁺/LAG-3⁻ T cells,
   (m) CD8⁺/4-1BB⁺/LAG-3⁺ T cells,
   (n) CD8⁺/4-1BB⁻/LAG-3⁺ T cells,
   (o) CD8⁺/4-IBB⁺/LAG-3⁻ T cells,
   (p) CD8⁺/4-1BB⁺/TIM-3⁺ T cells,
   (q) CD8⁺/4-1BB⁻/TIM-3⁺ T cells, and
   (r) CD8⁺/4-1BB⁺/TIM-3⁻ T cells,
   wherein the cell population is enriched for tumor-reactive T cells.
21. The isolated or purified cell population of embodiment 20 comprising:
   (a) CD8⁺/4-1BB⁺/PD-1⁺ T cells,
   (b) CD8⁺/4-1BB⁻/PD-1⁺ T cells,
   (c) CD8⁺/4-1BB⁺/PD-1T cells,
   (d) CD8⁺/LAG-3⁺/PD-1⁺ T cells,
   (e) CD8⁺/LAG-3⁻/PD-1⁺ T cells,
   (f) CD8⁺/LAG-3⁺/PD-1⁻ T cells,
   (g) CD8⁺/TIM-3⁺/PD-1⁺ T cells,
   (h) CD8⁺/TIM-3⁻/PD-1⁺ T cells,
   (i) CD8⁺/TIM-3⁺/PD-1⁻ T cells,
   (j) CD8⁺/TIM-3⁺/LAG-3⁺ T cells,
   (k) CD8⁺/TIM-3⁻/LAG-3⁺ T cells,
   (l) CD8⁺/TIM-3⁺/LAG-3⁻ T cells,
   (m) CD8⁺/4-1BB⁺/LAG-3⁺ T cells,
   (n) CD8⁺/4-1BB⁻/LAG-3⁺ T cells,
   (o) CD8⁺/4-1BB⁺/LAG-3⁻ T cells,
   (p) CD8⁺/4-1BB⁺/TIM-3⁺ T cells,
   (q) CD8⁺/4-1BB⁻/TIM-3⁺ T cells, or
   (r) CD8⁺/4-1BB⁺/TIM-3⁻ T cells.
22. A cell population enriched for tumor-reactive T cells obtained by a method comprising:
   (a) obtaining a bulk population of T cells from a tumor sample;
   (b) specifically selecting CD8⁺ T cells that express any one or more of TIM-3, LAG-3, 4-1BB, and PD-1 from the bulk population; and
   (c) separating the cells selected in (b) from unselected cells to obtain a cell population enriched for tumor-reactive T cells,
   for use in administering the cell population enriched for tumor-reactive T cells to a mammal.
23. The cell population of embodiment 22, wherein (b) comprises specifically selecting CD8⁺ T cells that express TIM-3 from the bulk population.
24. The cell population of embodiment 22 or 23, wherein (b) comprises specifically selecting CD8⁺ T cells that express LAG-3 from the bulk population.
25. The cell population of any one of embodiments 22-24, wherein (b) comprises specifically selecting CD8⁺ T cells that express 4-1BB from the bulk population.
26. The cell population of any one of embodiments 22-25, wherein (b) comprises specifically selecting CD8⁺ T cells that express PD-1 from the bulk population.
27. The cell population of embodiment 22, wherein (b) comprises specifically selecting CD8⁺ T cells that are (i) 4-1BB⁺/PD-1⁺, (ii) 4-1BB/PD-1⁺, and/or (iii) 4-1BB⁺/PD-1⁻ from the bulk population.
28. The cell population of embodiment 22, wherein (b) comprises specifically selecting CD8⁺ T cells that are (i) LAG-3⁺/PD-1⁺, (ii) LAG-3⁻/PD-1⁺, and/or (iii) LAG-3⁺/PD-1⁻ from the bulk population.
29. The cell population of embodiment 22, wherein (b) comprises specifically selecting CD8⁺ T cells that are (i) TIM-3⁺/PD-1⁺, (ii) TIM-3⁻/PD-1⁺, or (iii) TIM-3⁺/PD-1⁻ from the bulk population.
30. The cell population of embodiment 22, wherein (b) comprises specifically selecting CD8⁺ T cells that are (i) TIM-3⁺/LAG-3⁺, (ii) TIM-3⁻/LAG-3⁺, or (iii) TIM-3⁺/LAG-3⁻ from the bulk population.
31. The cell population of embodiment 22, wherein (b) comprises specifically selecting CD8⁺ T cells that are (i) 4-1BB⁺/LAG-3⁺, (ii) 4-1BB⁻/LAG-3⁺, or (iii) 4-1BB⁺/LAG-3⁻ from the bulk population.
32. The cell population of embodiment 22, wherein (b) comprises specifically selecting CD8⁺ T cells that are (i) 4-1BB⁺/TIM-3⁺, (ii) 4-1BB⁻/TIM-3⁺, or (iii) 4-1BB⁺/TIM-3⁻ from the bulk population.
33. The cell population of any one of embodiments 22-32, wherein the cell population enriched for tumor-reactive T cells is obtained without screening for autologous tumor recognition.
34. The cell population of any one of embodiments 22-33, wherein the bulk population of T cells is not non-specifically stimulated prior to (b).
35. The cell population of any one of embodiment 22-34, further comprising expanding the numbers of T cells in the enriched cell population obtained in (c).
36. The cell population of any one of embodiments 22-35, further comprising culturing the enriched cell population obtained in (c) in the presence of any one or more of TWS119, interleukin (IL-21), IL-12, IL-15, IL-7, transforming growth factor (TGF) beta, and AKT inhibitor (AKTi).
37. The cell population of any one of embodiments 22-36, further comprising stimulating the enriched cell population obtained in (c) with a tumor antigen and/or with autologous tumor T cells.
38. The cell population of any one of embodiments 22-37, further comprising transducing or transfecting the cells of the enriched population obtained in (c) with a nucleotide sequence encoding any one or more of IL-12, IL-7, IL-15, IL-2, IL-21, mir155, andanti-PD-1 siRNA.
39. The cell population enriched for tumor-reactive T cells obtained by the method claimed in any one of embodiments 1 and 3-18, the pharmaceutical composition obtained by the method claimed in any one of embodiments 2-18, or the cell population of any one of embodiments 22-38, for use in treating or preventing cancer.

## Claims

1. A population of tumor-reactive T-cells for use as a medicament for treating cancer, wherein said population of cells is obtained by a method comprising:
(a) obtaining a bulk population of T cells from a tumor sample;
(b) specifically selecting CD8⁺ T cells that express any two or more of TIM-3, LAG-3, 4-1BB, and PD-1 from the bulk population; and
(c) separating the cells selected in (b) from unselected cells to obtain a cell population enriched for tumor-reactive T cells.

2. The population of tumor-reactive T-cells for the use of claim 1, wherein the method further comprises combining the cell population enriched for tumor-reactive T cells with a pharmaceutically acceptable carrier to obtain a pharmaceutical composition comprising the tumor-reactive T cells.

3. The population of tumor-reactive T-cells for the use of claim 1 or 2, wherein the method further comprises expanding the numbers of T-cells in the enriched cell population obtained in (c).

4. The population of tumor-reactive T-cells for the use of any one of claims 1-3, wherein the method further comprises culturing the enriched cell population obtained in (c) in the presence of any one or more of TWS119, interleukin (IL-21), IL-12, IL-15, IL-7, transforming growth factor (TGF) beta, and AKT inhibitor (AKTi).

5. The population of tumor-reactive T-cells for the use of any one of claims 1-4, wherein the method further comprises stimulating the enriched cell population obtained in (c) with a cancer antigen and/or with autologous tumor cells.

6. The population of tumor-reactive T-cells for the use of any one of claims 1-5, wherein the method further comprises transducing or transfecting the cells of the enriched population obtained in (c) with a nucleotide sequence encoding any one or more of IL-12, IL-7, IL-15, IL-2, IL-21, mir155, and anti-PD-1 siRNA.

7. The population of tumor-reactive T-cells for the use of any one of claims 1-6, wherein the method comprises selecting any one or more of the following cell types:
(a) CD8⁺/4-1BB⁺/PD-1⁺ T cells,
(b) CD8⁺/LAG-3⁺/PD-1⁺ T cells,
(c) CD8⁺/TIM-3⁺/PD-1⁺ T cells,
(d) CD8⁺/TIM-3⁺/LAG-3⁺ T cells,
(e) CD8⁺/4-1BB⁺/LAG-3⁺ T cells,
(f) CD8⁺/4-1BB⁺/TIM-3⁺ T cells,
(g) CD8⁺/4-1BB⁺/TIM-3⁺/LAG-3⁺ T cells,
(h) CD8⁺/4-1BB⁺/TIM-3⁺/PD-1⁺ T cells,
(i) CD8⁺/4-1BB⁺/LAG-3⁺/PD-1⁺ T cells,
(j) CD8⁺/LAG-3⁺/TIM-3⁺/PD-1⁺ T cells, and
(k) CD8⁺/4-1BB⁺/TIM-3⁺/LAG-3⁺/PD-1⁺ T cells,
wherein the cell population is enriched for tumor-reactive T cells.

8. A method of obtaining a cell population enriched for tumor-reactive T-cells, the method comprising:
(a) obtaining a bulk population of T cells from a tumor sample;
(b) specifically selecting CD8⁺ T cells that express three or more of TIM-3, LAG-3, 4-1BB, and PD-1 from the bulk population; and
(c) separating the cells selected in (b) from unselected cells to obtain a cell population enriched for tumor-reactive T cells.

9. A method of obtaining a pharmaceutical composition comprising a cell population enriched for tumor-reactive T cells, the method comprising:
(a) obtaining a bulk population of T cells from a tumor sample;
(b) specifically selecting CD8⁺ T cells that express three or more of TIM-3, LAG-3, 4-1BB, and PD-1 from the bulk population;
(c) separating the cells selected in (b) from unselected cells to obtain a cell population enriched for tumor-reactive T cells; and
(d) combining the cell population enriched for tumor-reactive T cells with a pharmaceutically acceptable carrier to obtain a pharmaceutical composition comprising a cell population enriched for tumor-reactive T cells.

10. The method of claim 8 or 9, wherein the method further comprises expanding the numbers of T-cells in the enriched cell population obtained in (c).

11. The method of any one of claims 8-10, wherein the method further comprises culturing the enriched cell population obtained in (c) in the presence of any one or more of TWS119, interleukin (IL-21), IL-12, IL-15, IL-7, transforming growth factor (TGF) beta, and AKT inhibitor (AKTi).

12. The method of any one of claims 8-11, wherein the method further comprises stimulating the enriched cell population obtained in (c) with a cancer antigen and/or with autologous tumor cells.

13. The method of any one of claims 8-12, wherein the method further comprises transducing or transfecting the cells of the enriched population obtained in (c) with a nucleotide sequence encoding any one or more of IL-12, IL-7, IL-15, IL-2, IL-21, mir155, and anti-PD-1 siRNA.

14. The method of any one of claims 8-13, wherein the method comprises selecting any one or more of the following cell types:
(a) CD8⁺/4-1BB⁺/TIM-3⁺/LAG-3⁺ T cells,
(b) CD8⁺/4-1BB⁺/TIM-3⁺/PD-1⁺ T cells,
(c) CD8⁺/4-1BB⁺/LAG-3⁺/PD-1⁺ T cells,
(d) CD8⁺/LAG-3⁺/TIM-3⁺/PD-1⁺ T cells, and
(e) CD8⁺/4-1BB⁺/TIM-3⁺/LAG-3⁺/PD-1⁺ T cells,
wherein the cell population is enriched for tumor-reactive T cells.
